# EUROPÄISCHE PATENTSCHRIFT

(11) **EP 0 686 621 B1**
(45) Veröffentlichungstag und Bekanntmachung des Hinweises auf die Patenterteilung: **11.03.1998**
(21) Anmeldenummer: 95108201.5
(22) Anmeldetag: 30.05.1995
(51) Int. Cl.: C07C 67/26, C07C 69/54, C08G 63/47, C07C 67/08

(54) **Verfahren zur Herstellung von strahlungshärtbaren Acrylaten und durch das Verfahren erhältliche Acrylate**
Process for the preparation of radiation-curable acrylates and acrylates obtainable therefrom
Procédé de préparation d'acrylates durcissables par des radiations et des acrylates obtenables par ce procédé

(30) Priorität: 08.06.1994 DE 4420012
(43) Veröffentlichungstag der Anmeldung: 13.12.1995
(73) Patentinhaber: BASF Aktiengesellschaft, 67063 Ludwigshafen (DE)
(72) Erfinder: Reich, Wolfgang, Dr., D-67133 Maxdorf (DE); Beck, Erich, Dr., D-69198 Schriesheim (DE); Keil, Edmund, D-67259 Heuchelheim (DE); Erhardt, Ulrich, Dr., D-68526 Ladenburg (DE); Nuber, Adolf, Dr., D-67459 Böhl-Iggelheim (DE)

(56) Entgegenhaltungen:
- EP-A- 0 279 303
- BE-A- 657 091
- US-A- 3 833 384

## Beschreibung

Die Erfindung betrifft ein Verfahren zur Herstellung von strahlungshärtbaren Acrylaten durch Umsetzung von Hydroxyverbindungen mit Acrylsäure oder Methacrylsäure. Aus der EP-A-54 105, der DE-A-33 16 593 und EP-A-279 303 sind Verfahren bekannt, bei denen in einer ersten Stufe ein (Meth)acrylsäureester aus (Meth)acrylsäure und einer Hydroxyverbindung hergestellt wird und in einer zweiten Stufe überschüssige (Meth)acrylsäure mit Epoxiden umgesetzt wird. Als Katalysatoren für die Umsetzung in der zweiten Stufe sind in der DE-A-33 16 593 tertiäre Amine oder Lewis Base wie Thiodiglycol genannt. In der EP-A-54 105 wird Triphenylphosphin als Katalysator eingesetzt. In der EP-A-279 303 werden neben tertiären Aminen und Lewisbasen in allgemeiner Form auch quartäre Ammoniumverbindungen genannt.

Die nach den bekannten Verfahren hergestellten strahlungshärtbaren Acrylate weisen einige Nachteile auf. So werden beim Zusatz von Isocyanatvernetzern, z.B. zur Erhöhung der Härte von Beschichtungen, strahlungshärtbare Zubereitungen mit ungenügender Lagerstabilität, d.h. nicht ausreichender Zeit für die Verarbeitung bis zum Einsetzen der Vernetzung, erhalten. Des weiteren ist beim Zusatz z.B. von Füllstoffen oder Pigmenten, zu den bisher bekannten strahlungshärtbaren Acrylaten ein unerwünschtes Absetzen von Bestandteilen der erhaltenen strahlungshärtbaren Zubereitung zu beobachten.

Aufgabe der vorliegenden Erfindung war daher ein Verfahren zur Herstellung von strahlungshärtbaren Acrylaten, welches diese Nachteile nicht aufweist.

Demgemäß wurde ein Verfahren zur Herstellung von strahlungshärtbaren Acrylaten durch Umsetzung von Hydroxyverbindungen mit Acrylsäure oder Methacrylsäure (1. Stufe), dadurch gekennzeichnet, daß das Reaktionsprodukt der 1. Stufe in einer 2. Stufe mit einer Epoxidverbindung in Gegenwart von quartären Ammonium- oder Phosphoniumverbindungen der allgemeinen Formeln 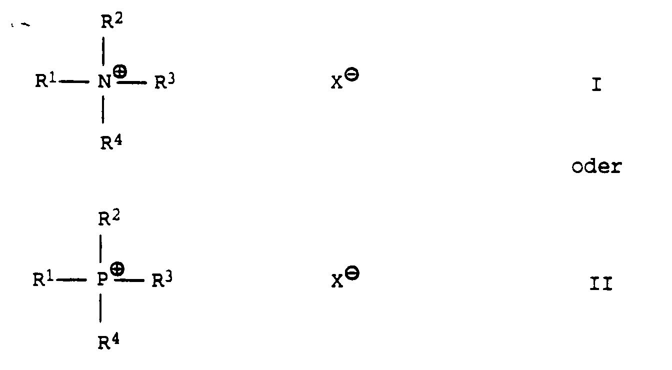 als Katalysatoren zur Reaktion gebracht wird, wobei X^{⊖} für ein Gegenion und R¹ bis R⁴ unabhängig voneinander für eine C₁-C₁₈-Alkylgruppe, welche gegebenenfalls durch ein oder zwei Phenylgruppen substituiert sein kann, oder eine C₆-C₁₂-Arylgruppe, welche gegebenenfalls durch ein oder zwei C₁-C₆-Alkylgrugpen substituiert sein kann, stehen.

Ebenfalls gefunden wurden die nach diesem Verfahren erhältlichen Acrylate sowie strahlungshärtbare Zubereitungen, welche diese Acrylate enthalten.

Beim erfindungsgemäßen Verfahren wird in einer 1. Stufe Acrylsäure oder Methacrylsäure (zusammenfassend (Meth)acrylsäure genannt) mit einer Hydroxyverbindung umgesetzt. Als Hydroxyverbindungen in Betracht kommen Verbindungen mit einer oder mehreren Hydroxygruppen. Genannt seien Monoalkohole, C₂-C₈-Alkylendiole. Trimethylolpropan, Glycerin oder Pentaerythrit oder z.B. mit Ethylenoxid oder Propylenoxid alkoxylierte, Hydroxygruppen enthaltende Verbindungen.

Bevorzugte Hydroxyverbindungen sind mindestens 2, insbesondere 2 bis 6 freie Hydroxylgruppen enthaltende, gesättigte Polyester, welche gegebenenfalls auch Ethergruppen enthalten können oder Polyether mit mindestens 2, insbesondere 2 bis 6 freien Hydroxylgruppen.

Die Molekulargewichte Mₙ der Polyester bzw. Polyether liegen bevorzugt zwischen 100 und 4000 (Mₙ bestimmt durch Gelpermeationschromatographie).

Derartige hydroxylgruppenhaltige Polyester können z.B. in üblicher Weise durch Veresterung von Dicarbonsäuren oder Polycarbonsäuren mit Diolen oder Polyolen hergestellt werden. Die Ausgangsstoffe für solche hydroxylgruppenhaltige Polyester sind dem Fachmann bekannt. Bevorzugt können als Dicarbonsäuren Bernsteinsäure, Glutarsäure, Adipinsäure, Sebacinsäure, o-Phthalsäure, deren Isomere und Hydrierungsprodukte sowie veresterbare Derivate, wie Anhydride, z.B. Maleinsäureanhydrid, oder Dialkylester der genannten Säuren eingesetzt werden. Als Polycarbonsäure kommt z.B. Trimellitsäure in Betracht. Als Diole kommen vorzugsweise in Betracht Ethylenglykol, Propylenglykol-1,2 und -1,3, Butandiol-1,4, Hexandiol-1,6, Neopentylglykol, Cyclohexandimethanol sowie Polyglykole vom Typ des Ethylenglykols und Propylenglykols.

Als Polyole sind in erster Linie Trimethylolpropan, Glycerin oder Pentaerythrit zu nennen.

Als Diole oder Polyole in Betracht kommen auch oxalkylierte (z.B. mit Ethylenoxid oder Propylenoxid) Diole oder Polyole, insbesondere mit einem Oxalkylierungsgrad von 0 bis 10, bezogen auf die jeweiligen Hydroxygruppen des Diols oder Polyols.

Zu den erfindungsgemäß einzusetzenden Polyesterolen zählen auch Polycaprolactondiole und -triole, deren Herstellung dem Fachmann ebenfalls bekannt ist.

Als hydroxylgruppenhaltige Polyether kommen z.B. solche in Frage, welche nach bekannten Verfahren durch Umsetzung von zwei- und/oder mehrwertigen Alkoholen mit verschiedenen Mengen an Ethylenoxid und/oder Propylenoxid erhalten werden können. Bei den Ethylenglykol/Propylenglykol-Mischkondensationsprodukten kann die Umsetzung zweckmäßigerweise so gesteuert werden, daß endständig überwiegend primäre Hydroxylgruppen entstehen. Desgleichen sind auch Polymerisationsprodukte des Tetrahydrofurans oder Butylenoxids verwendbar.

Bevorzugt sind Oxalkylierungsprodukte der obengenannten Diole oder Polyole, insbesondere mit einem Oxalkylierungsgrad von 0 bis 10, besonders bevorzugt von 1 bis 10, bezogen auf die jeweiligen Hydroxylgruppen des Diols oder Polyols, wobei jedoch insgesamt mindestens 2 Alkoxygruppen im Polyether vorhanden sind.

Bei der Veresterung der (Meth)acrylsäure im Falle des hydroxylgruppenhaltigen Polyesters ist es z.B. auch möglich, die (Meth)acrylsäure zusammen mit Ausgangsstoffen des hydroxylgruppenhaltigen Polyesters, z.B. Dicarbonsäuren oder deren Anhydride und Diole bzw. Polyole vorzulegen und die Ausgangsstoffe zusammen mit der (Meth)acrylsäure in einer Stufe umzusetzen.

Bei der Veresterung von (Meth)acrylsäure mit der Hydroxyverbindung werden bevorzugt 0,1 bis 1,5, besonders bevorzugt 0,5 bis 1,4 und ganz besonders bevorzugt 0,7 bis 1,3 Äquivalente (Meth)acrylsäure, bezogen auf 1 Hydroxy-Äquivalent der Hydroxyverbindungen eingesetzt. Im oben erwähnten Fall, daß auch Ausgangsstoffe z.B. des hydroxylgruppenenthaltenden Polyesters bei der Veresterung zugegen sind, beziehen sich die Aquivalente der (Meth)acrylsäure auf das theoretisch nach Reaktion der Ausgangsstoffe, z.B. Reaktion von Dicarbonsäuren mit Diolen oder Polyolen, verbleibende Hydroxyäquivalent.

Die Umsetzung der (Meth)acrylsäure mit den Hydroxyverbindungen kann z.B. in Gegenwart eines sauren Veresterungskatalysators, wie z.B. Schwefelsäure oder p-Toluolsulfonsäure, sowie in Gegenwart eines Kohlenwasserstoffs, der mit Wasser ein azeotropes Gemisch bildet, insbesondere bis zu einem Umsatz von insbesondere mindestens 85 %, vorzugsweise 90 bis 95 %, der Hydroxylgruppen der Hydroxyverbindung, beispielsweise bei 60 bis 140°C, durchgeführt werden. Das gebildete Reaktionswasser wird azeotrop entfernt. Geeignete Kohlenwasserstoffe sind aliphatische und aromatische, z.B. Alkane und Cycloalkane, wie n-Hexan, n-Heptan und Cyclohexan, Aromaten wie Benzol, Toluol und die Xylol-Isomeren, und sog. Spezialbenzine, welche Siedegrenzen zwischen 70 und 140°C aufweisen.

Zur Vermeidung einer vorzeitigen Polymerisation wird die Umsetzung mit (Meth)acrylsäure zweckmäßigerweise in Gegenwart geringer Mengen von Inhibitoren durchgeführt. Dabei handelt es sich um die üblichen, zur Verhinderung einer thermischen Polymerisation verwendeten Verbindungen, z.B. vom Typ des Hydrochinons, der Hydrochinonmonoalkylether, des 2,6-Di-t-butylphenols, der N-Nitrosoamine der Phenothiazine oder der Phophorigsäureester. Sie werden im allgemeinen in Mengen von 0,001 bis 2,0 %, vorzugsweise in Mengen von 0,005 bis 0,5 %, bezogen auf die Reaktion in der 1. Stufe eingesetzt.

Nach der Veresterung kann das Lösungsmittel, z.B. der Kohlenwasserstoff, aus dem Reaktionsgemisch destillativ, gegebenenfalls unter vermindertem Druck, entfernt werden. Der Veresterungskatalysator kann in geeigneter Weise neutralisiert werden, z.B. durch Zusatz von tertiären Aminen oder Alkalihydroxyden.

In der 2. Stufe wird das in der 1. Stufe erhaltene Reaktionsprodukt mit einer Epoxidverbindung umgesetzt. Epoxidverbindungen sind solche mit mindestens einer, bevorzugt mindestens zwei, vorzugsweise zwei oder drei Epoxidgruppen im Molekül.

In Betracht kommen z.B. epoxidierte Olefine, Glycidylester von gesättigten oder ungesättigten Carbonsäuren oder Glycidylether aliphatischer oder aromatische Polyole. Derartige Produkte werden im Handel in großer Zahl angeboten. Besonders bevorzugt sind Polyglycidylverbindungen von Bisphenol A-Typ und Glycidylether mehrfunktioneller Alkohole, z.B. des Butandiols, des Glycerins und des Pentaerythrits. Beispiele für derartige Polyepoxidverbindungen sind ®Epikote 812 (Epoxidwert: ca. 0,67) und Epikote 828 (Epoxidwert: ca. 0,53) und Epikote 162 (Epoxidwert: ca. 0,61) der Firma Shell.

Die Epoxidverbindungen werden dem in der 1. Stufe erhaltenen Reaktionsprodukt im allgemeinen in Mengen von 1 bis 20 Gew.-%, besonders bevorzugt von 5 bis 15 Gew.-%, bezogen auf das Reaktionsprodukt der 1. Stufe, zugegeben. Ganz besonders bevorzugt werden die Epoxidverbindungen in ungefähr äquimolaren Mengen, bezogen auf die noch vorhandenen Säureäquivalente im Reaktionsprodukt der 1. Stufe, eingesetzt.

Bei der Umsetzung mit Epoxidverbindungen in der 2. Stufe wird überschüssig eingesetzte bzw. nicht umgesetzte Säure, insbesondere (Meth)acrylsäure, daneben aber auch z.B. noch im Gemisch als Ausgangsstoff vorhandene Dicarbonsäure oder entstandene Halbester von Dicarbonsäuren mit einer verbleibenden Säufegruppe als Epoxidester gebunden.

Die Umsetzung mit Epoxidverbindungen erfolgt bevorzugt bei 90 bis 130, besonders bevorzugt bei 100 bis 110°C und wird vorzugsweise so lange durchgeführt, bis das Reaktionsgemisch eine Säurezahl unter 10, besonders bevorzugt unter 5 mg KOH/g aufweist.

Als Katalysator für die Umsetzung der Epoxidverbindungen mit den Säuregruppen in der 2. Stufe werden quartäre Ammonium- oder Phosphoniumverbindungen der allgemeinen Formeln 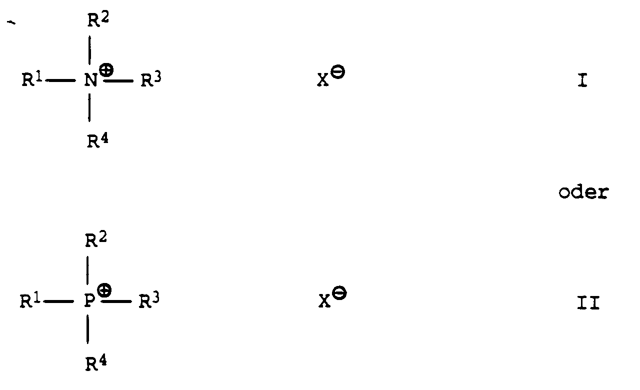 eingesetzt, wobei X^{⊖} für ein Gegenion und R¹ bis R⁴ unabhängig voneinander für eine C₁-C₁₈-Alkylgruppe, welche gegebenenfalls durch ein oder zwei Phenylgruppen substituiert sein kann, oder eine C₆-C₁₂-Arylgruppe, welche gegebenenfalls durch ein oder zwei C₁-C₆-Alkylgruppen sübstituiert sein kann, stehen.

Beispiele für Gegenionen X^{⊖} sind Br^{⊖}, Cl^{⊖}, J^{⊖}, HSO₄^{⊖}, OH^{⊖}, ClO₄^{⊖} , BF₄^{⊖}, Acetat.

Die Reste R¹ bis R⁴ stehen insbesondere für eine C₁-C₈-Alkylgruppe, vorzugsweise eine C₁-C₄-Alkylgruppe, oder eine Benzylgruppe.

Die Reste R¹ bis R⁴ können teilweise aliphatisch und teilweise aromatisch sein, insbesondere kann einer der Reste aromatisch sein, während die anderen drei Reste aliphatisch sind.

Besonders bevorzugt sind alle Reste R¹ bis R⁴ aliphatisch.

Insbesondere werden Phosphoniumverbindungen der Formel II eingesetzt.

Die quartären Ammonium- oder Phosphoniumverbindungen werden bevorzugt in Mengen von 0,01 bis 5, besonders bevorzugt von 0,1 bis 2 Gew.-%, bezogen auf die Epoxidverbindungen, eingesetzt.

Die nach dem erfindungsgemäßen Verfahren erhältlichen strahlungshärtbaren Acrylate eignen sich insbesondere zur Verwendung als Massen, welche thermisch, bevorzugt aber durch energiereiche Strahlung gehärtet werden können.

Sie können verwendet werden als bzw. in Beschichtungsmassen, z.B. Lacken, Druckfarben oder Klebstoffen, als Druckplatten, als Formkörper, zur Herstellung von Photoresisten, in der Stereolithographie oder als Gießmassen z.B. für optische Linsen.

Zur Verwendung als oder in Strahlungshärtbaren Massen können den Strahlungshärtbaren Acrylaten Zusatzstoffe wie Vernetzer, Verdicker, Verlaufsmittel oder Füllstoffe bzw. Pigmente etc. zugesetzt werden (im folgenden Strahlungshärtbare Zubereitung genannt).

Als Vernetzer für die nachträgliche Vernetzung kommen insbesondere Isocyanatverbindungen in Betracht. Geeignete Isocyanatverbindungen weisen mindestens 2 Isocyanatgruppen auf.

Geeignete Härter sind Polyisocyanate mit einer mittleren Isocyanat-Funktionalität von mindestens 2,0, bevorzugt von 2,2 bis 5,0 und einem Isocyanat-Gehalt von 5 bis 30 Gew.-%, bevorzugt von 10 bis 25 Gew.-%, die vorzugsweise eine maximale Viskosität von 10000 mPas bei 25°C aufweisen. Prinzipiell geeignet sind aliphatische, cycloaliphatische und aromatische Diisocyanate, z.B. 1,4-Butandiisocyanat, 1,6-Hexandiisocyanat, 2,2,4-und 2,4,4-Trimethylhexamethylendiisocyanat, Cyclohexandiisocyanat, Methylcyclohexandiisocyanat, Isophorondiisocyanat, 4,4'-Diisocyanatodiphenylmethan, 4,4'-Diisocyanatodicyclohexylmethan, 2,4- und 2,6-Toluylendiisocyanat, Tetramethylxylylendiisocyanat. Besonders geeignet sind auch Isocyanate oder Biurete der voranstehend genannten Diisocyanate.

Es können z.B. auch s.g. "Lackpolyisocyanate" eingesetzt, wie sie beispielsweise in der EP-PS 0 358 979 beschrieben sind. Bei diesen Verbindungen handelt es sich z.B. um die Uretdion-, Biuret- und Isocyanurat-Gruppen aufweisenden Additionsprodukte z.B. obiger Diisocyanaten wie z.B. 1,6-Diisocyanatohexan oder Isophorondiisocyanat, die eine geringere Viskosität von beispielsweise 50 bis 500 oder 50 bis 3000 mPas bei 25°C aufweisen können. Besonders bevorzugt sind Isocyanat-Härter, die zusätzlich noch eine die Dispergierbarkeit in Wasser gewährleistende Menge eines Emulgators enthalten, wobei der Emulgator ein Umsetzungsprodukt eines Polyisocyanats mit einem ein- oder mehrwertigen, nicht-ionischen Polyether-Alkohol mit mindestens einer, mindestens 10 Ethylenoxideinheiten aufweisenden Polyetherkette sein kann.

Derartige wasseremulgierbare Polyisocyanate sind beispielsweise in der EP-ES 0 206 059 oder der DE-OS 4 036 927 beschrieben.

Der Anteil der Isocyanatverbindungen als Vernetzer beträgt vorzugsweise 0,05 bis 30, besonders bevorzugt 0,5 bis 20 Gew.-%, bezogen auf die strahlungshärtbaren Acrylate.

Strahlungshärtbare Zubereitungen, welche die erfindungsgemäßen Acrylate und Isocyanatverbindungen enthalten, zeigen gegenüber entsprechenden Zubereitungen des Standes der Technik eine deutlich verbesserte Lagerstabilität, erkennbar am geringeren Anstieg der Viskosität im Laufe der Lagerzeit.

Als Pigmente und Füllstoffe, welche den strahlungshärtbaren Acrylaten oder strahlungshärtbaren Zubereitungen der strahlungshärtbaren Acrylate zugesetzt werden können, kommen z.B. anorganische oder organische Pigmente, Füllstoffe, wie Rutil, Anatas, Kreide, Talkum, BASO₄ in Betracht.

Der Anteil der Pigmente oder Füllstoffe beträgt im allgemeinen insgesamt 0 bis 70, vorzugsweise 1 bis 50 Gew.-%, bezogen auf die strahlungshärtbaren Acrylate.

Auch strahlungshärtbare Zubereitungen der erfindungsgemäßen Acrylatverbindungen, welche Pigmente oder Füllstoffe enthalten, zeigen eine verbesserte Lagerstabilität, erkennbar an dem kaum noch zu beobachtenden Absetzen von Bestandteilen der Zubereitung.

Die strahlungshärtbaren Acrylate bzw. ihre Zubereitungen können thermisch, vorzugsweise durch energiereiche Strahlung wie UV-Licht oder Elektronenstrahlen gehärtet werden.

Zur Strahlungshärtung durch UV-Licht werden üblicherweise Photoinitiatoren zugesetzt.

Als Photoinitiatoren in Betracht kommen z.B. Benzophenon und Derivate davon, wie z.B. Alkylbenzophenone, halogenmethylierte Benzophenone, Michlers Keton, sowie Benzoin und Benzoinether wie Ethylbenzoinether. Benzilketale wie Benzildimethylketal, Acetophenonderivate wie z.B. Hydroxy-2-methyl-1-phenylpropan-1-on und Hydroxycyclohexyl-phenylketon. Anthrachinon und seine Derivate wie Methylanthrachinon und insbesondere Acylphosphinoxide wie z.B. Lucirin® TPO (2,4,6-Trimethylbenzoyldiphenylphosphinoxid).

Die Photoinitiatoren, die je nach Verwendungszweck der erfindungsgemäßen Massen in Mengen zwischen 0,1 und 15 Gew.-%, vorzugsweise 1 bis 10 Gew.-%, bezogen auf die polymerisierbaren Komponenten, eingesetzt werden, können als einzelne Substanz oder, wegen häufiger vorteilhafter synergistischer Effekte, auch in Kombination miteinander verwendet werden.

### Beispiel

a) Herstellung des Polyesteracrylates
   In einer 2-1-Apparatur werden 694,6 g ethoxyliertes Trimethylolpropan mit einer OH-Zahl von 630 mg KOH/g, 190 g Adipinsäure, 327,3 g Methylcyclohexan, 424,5 g Acrylsäure und 6,5 g Schwefelsäure conc. in Anwesenheit geeigneter Stabilisatoren aufgeheizt. Innerhalb von 6,5 Stunden werden 156 g Wasser ausgekreist. Anschließend werden das Methylcyclohexan und überschüssige Acrylsäure bis zu einer Säurezahl (SZ) von 42,6 mg KOH/g Substanz im Vakuum entfernt.
   Der Ansatz wird geteilt:
   1. Auf 500 g Rohester werden 18,3 g Tetrabutylammoniumbromid (NBu₄Br) und 70,6 g Bisphenol-A-diglycidether bei einer Temperatur von 106 bis 108°C zugegeben. Nach 7 Stunden Reaktionsdauer wird das Produkt filtriert und abgefüllt.
      - SZ:: 0,2 mg KOH/g Substanz
      - Iodfarbzahl (IFZ):: 3 bis 4
      - Viskosität:: 3,1 Pas

      Anmerkung:
      NBu₄Br katalysiert besser als Tributylamin, so daß geringere Mengen an Katalysator und wegen der geringeren Nebenreaktionen auch weniger Epoxid nötig wären.
   2. Auf 500 g Rohester werden 10,5 g Tributylamin (äquimolar) und 70,6 g Bisphenol-A-diglycidether bei einer Temperatur von 106 bis 108°C zugegeben. Nach 7 Stunden Reaktionsdauer wird das Produkt filtriert und abgefüllt.
      - End-SZ:: 3,9 mg KOH/g Substanz
      - IFZ:: 5 bis 7
      - Viskosität:: 2,9 Pas
b) Verträglichkeit mit Isocyanaten
   Es werden je 100 Teile des Polyesteracrylates aus 1. und 2. mit 5 Teilen eines Polyisocyanates (Basonat® P LR 8781) versetzt und bei einer Temperatur von 60°C gelagert und Aussehen bzw. Viskositätsanstieg beobachtet.
   Das Harz aus Ansatz 1. war schon nach 14 Stunden vollständig geliert. Das Harz aus Ansatz 2. hatte nach 105 Stunden eine Viskosität von 5,8 Pas.

## Patentansprüche

1. Verfahren zur Herstellung von strahlungshärtbaren Acrylaten durch Umsetzung von Hydroxyverbindungen mit Acrylsäure oder Methacrylsäure (1. Stufe), dadurch gekennzeichnet, daß das Reaktionsprodukt der 1. Stufe in einer 2. Stufe mit einer Epoxidverbindung in Gegenwart von quartären Ammonium- oder Phosphoniumverbindungen der allgemeinen Formeln 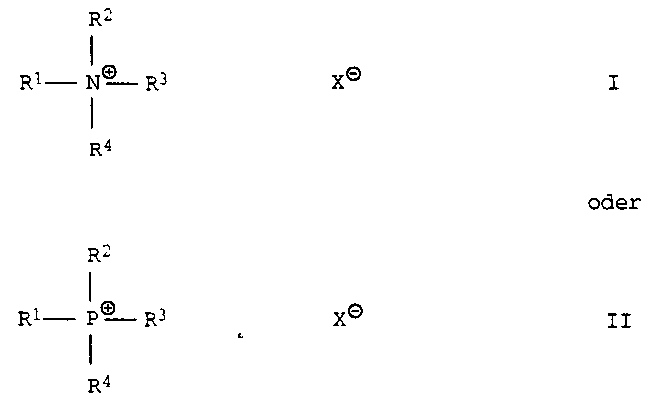 als Katalysatoren zur Reaktion gebracht wird, wobei X^{⊖} für ein Gegenion und R¹ bis R⁴ unabhängig voneinander für eine C₁-C₁₈-Alkylgruppe, welche gegebenenfalls durch ein oder zwei Phenylgruppen substituiert sein kann, oder eine C₆-C₁₂-Arylgruppe, welche gegebenenfalls durch ein oder zwei C₁-C₆-Alkylgruppen substituiert sein kann, stehen.

2. Verfahren nach Anspruch 1, dadurch gekennzeichnet, daß die Rest R¹ bis R⁴ unabhängig voneinander für C₁-C₈-Alkylgruppen stehen.

3. Verfahren nach Anspruch 1 oder 2, dadurch gekennzeichnet, daß Phosphoniumverbindungen der allgemeinen Formel II als Katalysatoren zugesetzt werden.

4. Verfahren nach einem der Ansprüche 1 bis 3, dadurch gekennzeichnet, daß es sich bei den Hydroxyverbindungen um gesättigte, mindestens zwei Hydroxygruppen im Molekül enthaltende Polyester, welche auch Ethergruppen enthalten können, oder um mindestens zwei Hydroxygruppen im Molekül enthaltende Polyether handelt.

5. Verfahren nach einem der Ansprüche 1 bis 4, dadurch gekennzeichnet, daß es sich bei den Epoxidverbindungen um Diepoxidverbindungen oder Triepoxidverbindungen handelt.

6. Strahlungshärtbare Acrylate, erhältlich durch ein Verfahren gemäß einem der Ansprüche 1 bis 5.

7. Strahlungshärtbare Zubereitung, enthaltend strahlungshärtbare Acrylate gemäß Anspruch 6 und zusätzlich eine Isocyanatverbindung mit mindestens zwei Isocyanatgruppen zur nachträglichen Vernetzung.

8. Strahlungshärtbare Zubereitung gemäß Anspruch 7, enthaltend 0,05 bis 30 Gew.-% der Isocyanatverbindung, bezogen auf die strahlungshärtbaren Acrylate.

9. Verwendung der strahlungshärtbaren Acrylate oder Zubereitungen gemäß einem der Ansprüche 6 bis 8 als strahlungshärtbare Masse.

## Claims

1. A process for the preparation of radiation-curable acrylates by reacting a hydroxy compound with acrylic acid or methacrylic acid (first stage), which comprises, in a second stage, reacting the reaction product of the first stage with an epoxide compound in the presence of, as a catalyst, a quaternary ammonium or phosphonium compound of the formula 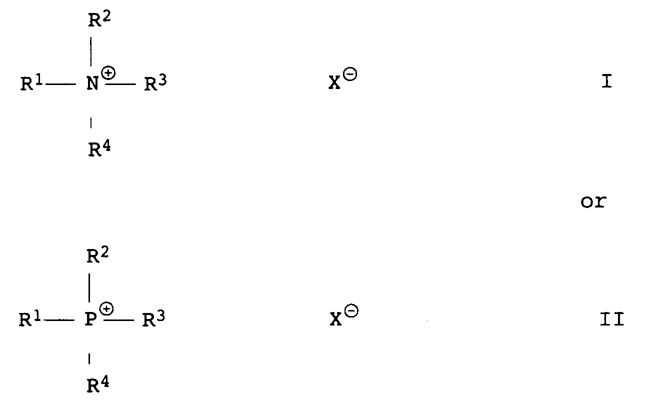 where X^{⊖} is an opposite ion and R¹ to R⁴ independently of one another are each C₁-C₁₈-alkyl which may be substituted by one or two phenyl groups or are each C₆-C₁₂-aryl which may be substituted by one or two C₁-C₆-alkyl groups.

2. A process as claimed in claim 1, wherein R¹ to R⁴ independently of one another are each C₁-C₈-alkyl.

3. A process as claimed in claim 1 or 2, wherein a phosphonium compound of the formula II is added as a catalyst.

4. A process as claimed in any of claims 1 to 3, wherein the hydroxy compound is a saturated polyester which contains at least two hydroxyl groups in the molecule and may also contain ether groups, or is a polyether containing at least two hydroxyl groups in the molecule.

5. A process as claimed in any of claims 1 to 4, wherein the epoxide compound is a diepoxide compound or triepoxide compound.

6. A radiation-curable acrylate obtainable by a process as claimed in any of claims 1 to 5.

7. A radiation-curable formulation containing a radiation-curable acrylate as claimed in claim 6 and in addition an isocyanate compound containing at least two isocyanate groups for subsequent crosslinking.

8. A radiation-curable formulation as claimed in claim 7, containing from 0.05 to 30% by weight, based on the radiation-curable acrylate, of the isocyanate compound.

9. The use of a radiation-curable acrylate or formulation as claimed in any of claims 6 to 8 as a radiation-curable material.

## Revendications

1. Procédé de préparation d'acrylates durcissables par irradiation par réaction de composés hydroxylés avec de l'acide acrylique ou de l'acide méthacrylique (1ère étape), caractérisé en ce que le produit de réaction de la 1ère étape est coupé dans une 2ème étape avec un composé époxy en présence de dérivés quaternaires d'ammonium ou de phosphonium de formule générale 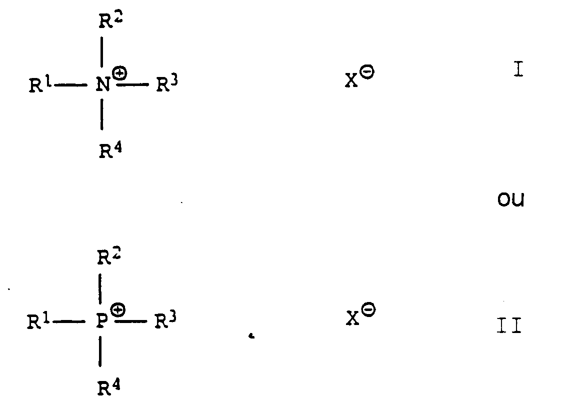 en tant que catalyseurs de la réaction, X Θ étant mis pour un contre-ion et R¹ à R⁴, indépendamment les uns des autres, pour un groupement alkyle en C₁-C₁₈ qui peut éventuellement être substitué par un ou deux groupements phényle, ou pour un groupement aryle en C₆-C₁₂ qui peut éventuellement être substitué par un ou deux groupements alkyle en C₁-C₆.

2. Procédé selon la revendication 1, caractérisé en ce que les restes R¹ à R⁴, indépendamment les uns des autres, sont mis pour des groupements alkyle en C₁-C₈.

3. Procédé selon l'une ou l'autre des revendications 1 et 2, caractérisé en ce qu'on utilise comme catalyseurs des dérivés de phosphonium de formule générale II.

4. Procédé selon l'une quelconque des revendications 1 à 3, caractérisé en ce que, pour les composés hydroxylés, il s'agit de polyester saturé contenant dans sa molécule au moins deux groupements hydroxy, lequel peut contenir aussi des groupements éther, ou de polyéther contenant dans sa molécule au moins deux groupements hydroxy.

5. Procédé selon l'une quelconque des revendications 1 à 4, caractérisé en ce que, pour les composés époxy, il s'agit de composés diépoxy ou de composés triépoxy.

6. Acrylates durcissables par irradiation, pouvant être obtenus par un procédé selon l'une des revendications 1 à 5.

7. Apprêt durcissable par irradiation, contenant un acrylate durcissable par irradiation selon la revendication 6 et également un composé isocyanate avec au moins deux groupements isocyanate pour une réticulation complémentaire.

8. Apprêt durcissable par irradiation selon la revendication 7, contenant 0,05 à 30% du dérivé isocyanate par rapport à l'acrylate durcissable par irradiation.

9. Utilisation de l'acrylate ou de l'apprêt durcissable par irradiation selon l'une quelconque des revendications 6 à 8 comme masse durcissable par irradiation.
